# EUROPÄISCHE PATENTANMELDUNG

(11) **EP 4 501 368 A1**
(43) Veröffentlichungstag der Anmeldung: **05.02.2025**
(21) Anmeldenummer: 24190224.6
(22) Anmeldetag: 23.07.2024
(51) Int. Cl.: A61M 1/02, A61M 39/20, A61M 5/178, A61P 7/06

(54) **VORRICHTUNG ZUR ABFÜLLUNG VON BLUTPRODUKTEN**

(30) Priorität: 02.08.2023 DE 202023104370 U
(71) Anmelder: Heinz Meise GmbH, 58579 Schalksmühle (DE)
(72) Erfinder: Meise, Heinz, 58579 Schalksmühle (DE)
(74) Vertreter: Patentanwälte Dörner & Kötter PartG mbB

(57) **Zusammenfassung**

Die Erfindung betrifft eine Vorrichtung zur Abfüllung von Blutprodukten in mindestens zwei Verabreichungsbehältnisse (3), umfassend einen Sammelbehälter (1), der über eine Zuführleitung (2) mit den Verabreichungsbehältnissen (3) verbunden ist, wobei die Verabreichungsbehältnisse (3) jeweils mit Öffnungsmitteln (31) versehen sind und durchströmbar miteinander verbunden sind, wobei die Verabreichungsbehältnisse (3) auf nur einer Seite durch Schläuche (35) miteinander verbunden sind. Die Öffnungsmittel (31) sind dabei zerstörungsfrei betätigbar.

## Beschreibung

Die Erfindung betrifft eine Vorrichtung zur Abfüllung von Blutprodukten in mindestens zwei Verabreichungsbehältnisse, umfassend einen Sammelbehälter, der über eine Zuführleitung mit den Verabreichungsbehältnissen verbunden ist, wobei die Verabreichungsbehältnisse jeweils mit Öffnungsmitteln versehen sind und durchströmbar miteinander verbunden sind, wobei die Verabreichungsbehältnisse auf nur einer Seite durch Schläuche miteinander verbunden sind.

Eine Vorrichtung zur Abfüllung von Blutprodukten ist beispielsweise aus der DE 10 2009 022 793 A1 bekannt. Bei dieser wird über ein Entnahmemittel dem jeweiligen Spender Blut entnommen, welches in den Sammel- und Trennbehälter geleitet wird. Von dort gelangt das Blut beziehungsweise das abgetrennte Blutprodukt über die Zufuhrleitung in die Verabreichungsbehältnisse. Die Verabreichungsbehältnisse sind mit Öffnungsmitteln versehen, die eine Entnahme des Blutproduktes aus dem jeweiligen Verabreichungsbehältnis ermöglichen.

Bei der bekannten Vorrichtung sind die Verabreichungsbehältnisse durchströmbar miteinander verbunden. Die Verbindung ist durch Leitungen geschaffen, die zwischen den benachbarten Verabreichungsbehältnissen angeordnet sind. Dabei sind die Leitungen abwechselnd in den den Öffnungsmitteln zugewandten und den den Öffnungsmitteln abgewandten Bereich - also oben und unten - an den Verabreichungsbehältnissen vorgesehen. Durch diese Ausbildung füllen sich jeweils diejenigen benachbarten Verabreichungsbehältnisse gleichmäßig, die über dem Boden zugewandte Leitungen miteinander verbunden sind. In Folge dessen ist der Füllstand dieser beiden zueinander benachbarten Verabreichungsbehältnisse jeweils gleich. Erst nach vollständiger Anfüllung dieser beiden Verabreichungsbehältnisse erfolgt ein Überlauf durch die dem Boden abgewandte - obere - Leitung zwischen den Verabreichungsbehältnissen. In Folge dessen werden immer zwei Behältnisse gleichmäßig gefüllt. Erst nach vollständiger Füllung der beiden Behältnisse erfolgt die Befüllung der beiden benachbarten Behältnisse. Dies hat zur Folge, dass bei vollständiger Entleerung des Sammel- und Trennbehälters die Gefahr einer lediglich teilweisen Anfüllung der beiden zuletzt angeströmten Verabreichungsbehältnisse besteht, obwohl die Menge des in diesen Behältnissen vorgesehenen Blutproduktes ausreichend sein kann, um ein Verabreichungsbehältnis vollständig anzufüllen. Da die Gewinnung der Blutprodukte kostenintensiv ist, führt diese Ausgestaltung zu einer Verschwendung von gewonnenen Blutprodukten.

Zur Beseitigung dieses Nachteils ist aus der DE 20 2011 004 487 U1 eine Vorrichtung bekannt, bei der die Verabreichungsbehältnisse auf nur einer Seite durch Schläuche miteinander verbunden sind. Dadurch ist eine Befüllung der Verabreichungsbehältnisse ermöglicht, bei der die Befüllung des benachbarten Behältnisses erst erfolgt, wenn das zuvor durchströmte Behältnis vollständig gefüllt ist. Auf diese Weise ist die nicht zu nutzende Menge an gewonnenem Blutprodukt aufgrund nicht vollständiger Befüllung des Verabreichungsbehältnisses wesentlich reduziert.

Die bekannten Vorrichtungen erfüllen die an Sie gestellten Anforderungen. Allerdings sind die Verabreichungsbehältnisse durchweg mit nicht wieder verschließbaren Öffnungsmitteln versehen. Das hat zur Folge, dass nach einem Öffnen eines Verabreichungsbehältnisses das darin befindliche Blutprodukt vollständig verabreicht werden muss, um keine Verschwendung zu provozieren. Dies ist jedoch vielfach nicht notwendig, da je nach Anwendungsfall lediglich geringe Mengen des Blutprodukts verabreicht werden müssen.

Vor diesem Hintergrund liegt der Erfindung die Aufgabe zugrunde, eine Vorrichtung zur Abfüllung von Blutprodukten in mindestens zwei Verabreichungsbehältnisse zu schaffen, bei der eine nur teilweise Entnahme des Blutproduktes aus einem Verabreichungsmittel mit gleichzeitig sterilem Wiederverschließen des Verabreichungsbehältnisses möglich ist. Gemäß der Erfindung wird diese Aufgabe durch die Merkmale des Patentanspruchs 1 gelöst.

Mit der Erfindung eine Vorrichtung zur Abfüllung von Blutprodukten in mindestens zwei Verabreichungsbehältnisse geschaffen, bei der eine nur teilweise Entnahme des Blutproduktes aus einem Verabreichungsmittel mit gleichzeitig sterilem Wiederverschließen des Verabreichungsbehältnisses möglich ist. Durch die Verwendung der sterilen und selbstdichtenden Adapter an den Verabreichungsmitteln besteht die Möglichkeit, die notwendige Menge des Blutproduktes mit Hilfe einer Spritze zu entnehmen. Hierzu wird die Kanüle der Spritze durch den Adapter in das Verabreichungsmittel eingeführt. Durch Aufziehen der Spritze wird die gewünschte Menge des Blutproduktes entnommen. Nach der Entnahme wird die Kanüle der Spritze aus dem Adapter herausgezogen. Der Adapter dichtet dabei selbsttätig und steril ab.

In Weiterbildung der Erfindung bestehen die Öffnungsmittel aus einem Anschusskonnektor, die in die Öffnung der Verabreichungsbehältnisse eingedrückt sind. Diese Gestaltung der Öffnungsmittel ist technisch einfach und zugleich zuverlässig. Zudem lassen sich die Anschlusskonnektoren einfach montieren.

Vorteilhaft sind die Anschlusskonnektoren aus Gummi hergestellt. Die Herstellung aus Gummi ist zum einen preiswert. Zum anderen ist durch Auswahl dieses Materials das selbstständige Verschließen der Verabreichungsbehältnisse zuverlässig gewährleistet.

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben. Es zeigen:

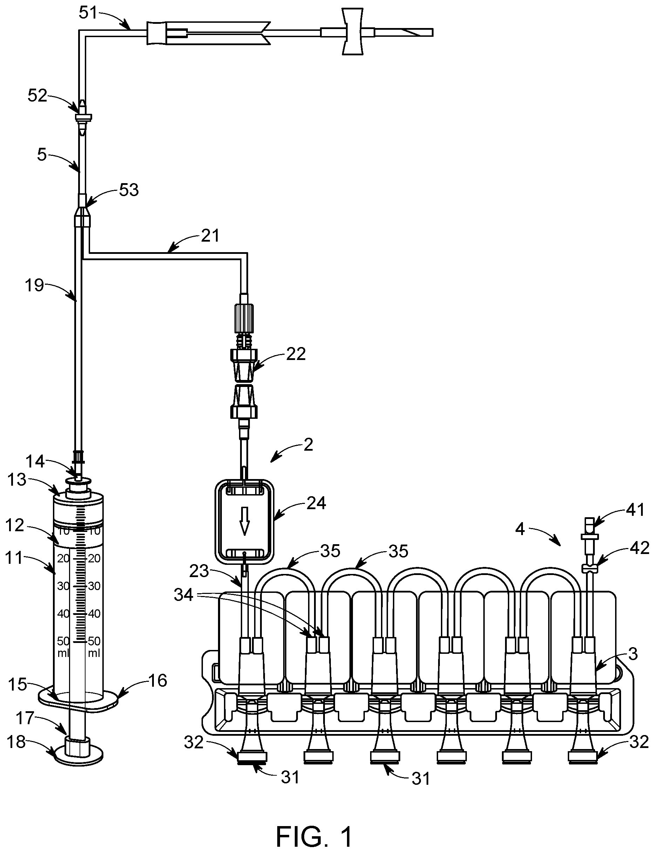

- Figur 1: die schematische Darstellung einer Vorrichtung zur Abfüllung von Blutprodukten mit Verabreichungsbehältnissen und Sammelbehälter.

Die als Ausführungsbeispiel gewählte Vorrichtung zur Abfüllung von Blutprodukten umfasst einen Sammelbehälter 1, der über eine Zuführleitung 2 mit Verabreichungsbehältnissen 3 verbunden ist.

Im Ausführungsbeispiel sind vier Verabreichungsbehältnisse 3 vorgesehen, die in Reihe miteinander verbunden sind. Die Entlüftung der Vorrichtung erfolgt über eine Endleitung 4. Die Verbindung zum Spender ist über eine Spenderleitung 5 geschaffen.

Der Sammelbehälter 1 ist im Ausführungsbeispiel nach Art einer Spritze ausgeführt. Andere Ausgestaltungen, beispielsweise in Form eines Beutels sind selbstverständlich ebenfalls möglich. Die Spritze hat im Ausführungsbeispiel ein Fassungsvermögen von 50 ml. Andere Größen sind möglich. Sie ist aus Glas, Kunststoff o. dgl. hergestellt. Mit der Spritze kann dem Patienten Blut entnommen werden.

Die Spritze besteht aus einem hohlen, zylinderförmigen Mantel 11, in dem ein beweglicher Kolben 12 auf- und abgleiten kann. An einem Ende ist der Spritzenmantel 11 durch eine Deckplatte 13 verschlossen, die in einem vorne offenen Konus 14, der Spritzendüse ausläuft. An den Konus 14 ist ein Schlauchstück 19 angeschlossen. Auf der Außenwand des Mantels 11 ist eine nicht dargestellte Skala angebracht, auf der man das jeweilige Injektions- oder Entnahmevolumen ablesen kann.

An dem der Deckplatte 13 abgewandten Ende des Spritzenmantels 11 befindet sich innen ein Kolbenstopp 15, um das Herausgleiten des Kolbens 12 zu verhindern. Außen weist der Spritzenmantels 11 in diesem Bereich eine Fingerauflage 16 auf. An dem Kolben 12 ist eine Kolbenstange 17 befestigt, die das der Deckplatte 13 abgewandten Ende des Spritzenmantels 11 durchsetzt. Am freien Ende der Kolbenstange 17 ist eine Daumenauflage 18 ausgebildet.

Die Zuführleitung 2 weist einen ersten Schlauchabschnitt 21 auf, der in einem ersten Teil eines Sterilkonnektors 22 mündet. Aufbau und Funktionsweise des Sterilkonnektors 22 sind bspw. in der DE 20 2009 008 274 U1 oder DE 20 2014 100 585 U1 beschrieben.

Der zweite Teil des Sterilkonnektors 22 ist an einem weiteren Leitungsabschnitt 23 angeordnet, der in einem der Verabreichungsbehältnisse 3 mündet. In dem Leitungsabschnitt 23 ist ein Filter 24 zur Zelltrennung angeordnet.

Die Verabreichungsbehältnisse 3 sind in Form kleiner Fläschchen aus Kunststoff ausgebildet. Sie haben im Ausführungsbeispiel ein Fassungsvermögen von 1,5 ml. Die Verabreichungsbehältnisse 3 sind jeweils mit Öffnungsmitteln 31 versehen. Die Öffnungsmittel 31 sind zerstörungsfrei zu betätigen. Die Öffnungsmittel 31 bestehen aus einem Anschusskonnektor, der in die Öffnung des jeweiligen Behältnisses eingedrückt ist und die Öffnung flüssigkeitsdicht verschließt. Der Anschlusskonnektor ist aus Gummi hergestellt. Der Anschlusskonnektor weist an seinen radial äußeren Enden ein Ringelement 32 aus Aluminium auf.

Auf der dem Öffnungsmittel 31 abgewandten Seite ist jedes Verabreichungsbehältnis 3 jeweils mit zwei Öffnungen 34 versehen. An die Öffnungen 34 sind Schläuche 35 angeschlossen, mit deren Hilfe die Verabreichungsbehältnisse 3 miteinander verbunden sind. Erkennbar sind die Verabreichungsbehältnisse 3 nur auf einer Seite durch die Schläuche 35 miteinander verbunden. Die Schläuche 35 sind im Wesentlichen senkrecht in die Verabreichungsbehältnisse 3 herein- und herausgeführt. An dem äußersten und in Durchströmrichtung ersten Verabreichungsbehältnis 3 ist an der ersten Öffnung 34 die Zuführleitung 2 angeschlossen. An die zweite Öffnung 35 des letzten Verabreichungsbehältnisses 3 ist ein Endschlauch 4 angeschlossen, der einen sog. Self-Sealing-Filter 41 aufweist. Der Endschlauch 4 mündet in einen nicht dargestellten Auffang- und Entlüftungsbehälter, bei dem es sich in der Regel um einen Kunststoffbeutel handelt.

Die Spenderleitung 5 weist an ihrem der Vorrichtung abgewandten Ende einen nicht dargestellten Anschluss zur Verbindung mit einem üblichen Zugang auf. Der Anschluss ist an einem Schlauchstück 51 angeordnet, welches ein Rückschlagventil 52 aufweist.

Das Schlauchstück 51 mündet in einen Y-Konnektor 53, an den außerdem sowohl das Schlauchstück 19 als auch der Schlauchabschnitt 21 angeschlossen sind.

Bei der Verwendung der erfindungsgemäßen Vorrichtung erfolgt zunächst die Blutentnahme bei dem jeweiligen Spender. Hierzu wird dem Spender in bekannter Weise ein Zugang gelegt. Die Entnahme kann mit einer Standard-Kanüle erfolgen. Beim Aufziehen des Kolbens 12 wird das Blut über das Schlauchstück 51 durch das Rückschlagventil 52 sowie den Y-Konnektor 53 und das Schlauchstück 19 in dem Sammelbehälter 1 aufgefangen. Anschließend wird das Schlauchstück 51 mit Hilfe einer nicht dargestellten Schlauchklemme geschlossen. Die Kanüle wird im Anschluss von dem Sammelbehälter 1 durch Durchtrennen des Schlauchstücks 51 getrennt. Der Behälter 1 wird nach der Befüllung ca. 2 Stunden aufrecht stehend gelagert. In dieser Zeit setzen sich Blutklumpen am Boden des Behälters 1 ab, während sich im oberen Bereich das gewünschte Blutprodukt bzw. das Serum sammelt.

Im Anschluss werden die Verabreichungsbehältnisse 3 mit Hilfe des Sterilkonnektors 22 an den Schlauchabschnitt 21 und damit an den Y-Konnektor 53 angeschlossen. Über das ebenfalls am Y-Konnektor 53 angeschlossene Schlauchstück 19 ist so die Verbindung zum Sammelbehälter 1 hergestellt.

Die Befüllung der Vorrichtung erfolgt durch Drücken auf den die Daumenauflage 18, wodurch über die Verbindung mit der Kolbenstange 17 der Kolben 12 in den Behälter 1 einfährt, wodurch das Serum, welches sich in dem dem Konus 14 zugewandten Bereich abgesetzt hat, aus dem Behälter 1 gedrückt wird. Das Blutprodukt tritt durch den Konus 14 aus dem Behälter 1 aus. Es durchströmt dann das Schlauchstück 19 und den Y-Konnektor 53 und tritt in das Schlauchstück 51 ein. Aufgrund des Rückschlagventils 52 ist ein weiteres Durchströmen verhindert. Vielmehr wird weiter aus dem Behälter 1 austretendes Blutprodukt im Y-Konnektor 53 umgelenkt in Richtung des Schlauchabschnitts 21 der Zuführleitung 2.

Nach Durchströmen der Zuführleitung 2 tritt das Blutprodukt durch eine der beiden Öffnungen 34 in das erste Verabreichungsbehältnis 3 ein. Nach vollständiger Befüllung des ersten Verabreichungsbehältnisses 3 steigt bei weiterer Zufuhr das Niveau des Blutproduktes in dem ersten Verabreichungsbehältnis 3 durch die weitere Öffnung 34 in den daran angeschlossenen Schlauch 35 an und läuft bei weiterer Befüllung des ersten Verabreichungsbehältnisses 3 über in das zweite Verabreichungsbehältnis 3. Auch hier erfolgt eine vollständige Befüllung mit Blutprodukt, so dass sich im Anschluss auch hier das Blutprodukt durch die zweite Öffnung 34 in dem zweiten Verabreichungsbehältnis 3 in den daran angeschlossenen Schlauch 35 erstreckt, bis das Blutprodukt in das benachbarte dritte Verabreichungsbehältnis eintritt. Dieser Vorgang setzt sich fort, bis das vorher gewonnene Blutprodukt aufgebraucht ist oder alle angeschlossenen Verabreichungsbehältnisse 3 vollständig gefüllt sind.

Durch die sequentielle, also der Reihe nach erfolgende Befüllung der einzelnen Verabreichungsbehältnisse ist gewährleistet, dass ein Übertritt des Blutproduktes in das benachbarte Verabreichungsbehältnis erst erfolgt, wenn das vorherige Verabreichungsbehältnis vollständig gefüllt ist. Somit besteht nicht die Gefahr, dass bei vollständiger Entleerung des Sammelbehälters am Ende mehrere Verabreichungsbehältnisse lediglich teilweise mit dem Blutprodukt gefüllt sind. Folglich ist eine optimale Ausnutzung des in dem Sammelbehälter vorgesehenen Blutproduktes hervorgerufen.

Nach der vollständigen Befüllung aller Verabreichungsbehältnisse 3 werden diese von dem Sammelbehälter 1 getrennt, indem die Zuführleitung 2 im Bereich des zweiten Leitungsabschnitts 23 mit Hilfe einer Thermo-Schweißzange durchtrennt wird. Die Leitung ist danach steril verschlossen. Ebenso wird der Endschlauch 4 mit Hilfe der Thermo-Schweißzange durchtrennt und ist dann mittels eines Schweißpunktes 42 verschlossen. Zudem kann eine Trennung der zueinander benachbarten Verabreichungsbehältnisse 3 erfolgen, so dass jedes Verabreichungsbehältnis 3 einzeln durch Betätigung der Öffnungsmitteln 31 verwendbar ist.

Zur Entnahme des Blutproduktes bzw. Serums wird auf den Konus einer üblichen medizinischen Spritze eine Injektionsnadel aufgesetzt, welche einen Durchmesser von wenigen Millimetern hat. Die Injektionsnadel wird dann durch den aus Gummi bestehenden Anschusskonnektor des Öffnungsmittels 31 gestochen. Über das Aufziehen des Kolbens der Spritze kann eine gewünschte Menge des Blutproduktes in die Spritze gezogen werden. Wenn sich die gewünschte Menge in der Spritze befindet, kann die Injektionsnadel wieder aus dem Anschusskonnektor des Öffnungsmittels 31 herausgezogen. Aufgrund der Materialeigenschaften des Anschlusskonnektors verschließt sich das durch die Injektionsnadel hervorgerufene sehr kleine Loch im Anschlusskonnektor selbstständig, sodass das Öffnungsmittel 31 wieder verschlossen ist. Soweit sich nach der Entnahme noch Reste des Blutproduktes in dem Verabreichungsbehältnis 3 befinden, können diese auf die gleiche Weise zu einem späteren Zeitpunkt entnommen werden.

Anders als im Stand der Technik kann bei der erfindungsgemäßen Vorrichtung mehrfach Serum bzw. das Blutprodukt aus den Verabreichungsbehältnissen entnommen werden. Dadurch ist gewährleistet, dass eine vollständige Entleerung der Verabreichungsbehältnisse erfolgt. Gleichzeitig ist eine Verschwendung vermieden, weil eine bedarfsgerechte Menge entnommen werden kann. Bei den aus dem Stand der Technik bekannten Vorrichtungen ist ein Verschlie-ßen der Verabreichungsbehältnisse nicht möglich. In Folge dessen erfolgt unter Umständen eine Entsorgung von Blutprodukt, wenn die Menge des Blutproduktes in dem Verabreichungsmittel größer ist als die Menge, die dem Patienten verabreicht werden soll. Dies führt zu einer Verschwendung von gewonnenen Blutprodukten, die bei der erfindungsgemäßen Vorrichtung vermieden ist.

## Patentansprüche

1. Vorrichtung zur Abfüllung von Blutprodukten in mindestens zwei Verabreichungsbehältnisse (3), umfassend einen Sammelbehälter (1), der über eine Zuführleitung (2) mit den Verabreichungsbehältnissen (3) verbunden ist, wobei die Verabreichungsbehältnisse (3) jeweils mit Öffnungsmitteln (31) versehen sind und durchströmbar miteinander verbunden sind, wobei die Verabreichungsbehältnisse (3) auf nur einer Seite durch Schläuche (35) miteinander verbunden sind, **dadurch gekennzeichnet, dass** die Öffnungsmittel (31) zerstörungsfrei betätigbar sind.

2. Vorrichtung nach Anspruch 1, **dadurch gekennzeichnet, dass** die Öffnungsmittel (31) aus einem Anschusskonnektor bestehen, die in die Öffnung der Verabreichungsbehältnisse (3) eingedrückt sind.

3. Vorrichtung nach Anspruch 2, **dadurch gekennzeichnet, dass** die Anschlusskonnektoren aus Gummi hergestellt sind.

4. Vorrichtung nach Anspruch 2 oder 3, **dadurch gekennzeichnet, dass** die Anschlusskonnektoren an ihren radial äußeren Enden ein Ringelement (32) aus Aluminium aufweisen.

5. Vorrichtung nach einem der vorgenannten Ansprüche, **dadurch gekennzeichnet, dass** die Schläuche (35) auf der den Öffnungsmitteln (31) abgewandten Seite angeordnet sind.

6. Vorrichtung nach einem der vorgenannten Ansprüche, **dadurch gekennzeichnet, dass** die Verabreichungsbehältnisse (3) in Reihe angeordnet sind.
